# EUROPÄISCHE PATENTSCHRIFT

(11) **EP 2 895 216 B1**
(45) Veröffentlichungstag und Bekanntmachung des Hinweises auf die Patenterteilung: **06.03.2019**
(21) Anmeldenummer: 13766892.7
(22) Anmeldetag: 13.09.2013
(51) Int. Cl.: A61M 1/36, B29C 65/00, G01L 19/00, G01L 19/06

(54) **DRUCKWANDLER-SCHUTZVORRICHTUNG FÜR BLUTSCHLAUCHSYSTEME**
PRESSURE TRANSDUCER PROTECTION DEVICE FOR BLOOD LINE SYSTEMS
DISPOSITIF DE PROTECTION DE TRANSDUCTEUR DE PRESSION POUR SYSTÈMES DE TUBULURES SANGUINES

(30) Priorität: 13.09.2012 DE 102012018078; 13.09.2012 US 201261700549 P
(43) Veröffentlichungstag der Anmeldung: 22.07.2015
(73) Patentinhaber: Fresenius Medical Care Deutschland GmbH, 61352 Bad Homburg (DE)
(72) Erfinder: BRÜCKNER, Christoph, 97422 Schweinfurt (DE)
(74) Vertreter: Schön, Andrea
(86) Internationale Anmeldenummer: PCT/EP2013/002763
(87) Internationale Veröffentlichungsnummer: WO 2014/040743

(56) Entgegenhaltungen:
- EP-A1- 2 253 343
- US-A- 4 226 124
- US-A1- 2004 237 785
- US-A1- 2008 221 499

## Beschreibung

### Zusammenfassung

Die Erfindung betrifft das Gebiet der Schutzvorrichtungen für Druckwandler von Maschinen zur extrakorporalen Behandlung von Blut, wie z.B. Dialysemaschinen.

### Stand der Technik

Schutzvorrichtungen für Druckwandler von Dialysemaschinen sind bekannt z.B. aus der EP 0 878 628 B1, der EP 0 652 018 B1 und der EP 2 253 343 A1. In der US 2004/0237785 wird eine Wandlerschutzvorrichtung beschrieben, die zwei hintereinandergeschaltete Hydropmembranen ausweist. In der US 4,226,124 wird eine Wandlerschutzvorrichtung beschrieben, die durch eine speziell geformte Trennmembran eine besonders gute Druckübertragung zum Druckwandler bereitstellt.

### Aufgabenstellung

Bei Verfahren zur extrakorporalen Blutbehandlung, wie z. B. Hämodialyse, wird das Blut des Patienten von einer Behandlungsmaschine durch ein Blutschlauchsystem mit einem Behandlungsmittel, wie z.B. ein Dialysator, transportiert. In diesem extrakorporalen Blutkreislauf muss der Druck überwacht werden, um physiologische und verfahrenstechnische Parameter im extrakorporalen System zu überwachen eventuelle Patientenzugangsprobleme oder Verschlüsse im extrakorporalen System zu detektieren. Zur Überwachung des Drucks im extrakorporalen Kreislauf weisen Maschinen zur extrakorporalen Blutbehandlung einen Druckwandler auf, der an das Blutschlauchsystem angeschlossen wird. Um auf der einen Seite eine Kontamination des Gerätes durch Patientenblut zu verhindern und auf der anderen Seite die Sterilität des Blutkreislaufes zu gewährleisten, muss zwischen die Behandlungsmaschine und dem extrakorporalen Blutkreislauf eine gasdurchlässige Antikontaminationsbarriere angeordnet werden. Die dazu üblicherweise verwendeten Wandler-Schutzvorrichtungen bestehen aus einem zweiteiligen Kunststoffgehäuse. Diese weisen zwei rohrförmige coaxial angeordnete Verbindungsteile auf. Das erste Verbindungsteil dient zum Anschluss der Schutzvorrichtung an den maschinenseitigen Druckwandler. Das zweite Verbindungsteil ist mit dem Blutschlauchsystem verbunden. Beide Verbindungsteile weisen weiterhin einen radialen Flansch auf, über den die beiden Teile dauerhaft, dicht miteinander verbunden werden. Zwischen diesen Flanschen befindet sich eine gasdurchlässige Membran, die quer zwischen den rohrförmigen Anschlüssen angeordnet ist.

Zur Befestigung der Wandler-Schutzvorrichtung an der Dialysemaschine muss das Pflegepersonal das Gehäuse mit dem ersten Verbindungsteil in den mit dem Druckwandler verbundenen Stutzen an der Oberfläche der Maschine einschrauben. Dazu muss das Gehäuse am schmalen Rand dieses Flansches gefasst und in genau paralleler Ausrichtung des Flansches zur Oberfläche des Maschinengehäuses in die Verbindung zum Druckwandler eingedreht werden. Es steht nur eine kleine Grifffläche zur Verfügung und das Gehäuse ist auf einer Seite gleichzeitig noch mit dem Blutschlauchsystem verbunden, was dazu führt, das die handelsüblichen Wandler-Schutzvorrichtungen unhandlich sind und die Befestigung recht unbequem für den Anwender ist.

Ein weiteres Problem bei den handelsüblichen Wandler-Schutzvorrichtungen ist die schnelle und einfache Erkennung von Fehlerzuständen.

Läuft eine Behandlung ohne Probleme, ist zwischen dem Blutpegel im Schlauchsystem und der Wandler-Schutzvorrichtung immer eine genügend hohe Luftsäule vorhanden. Fehlerzustände können aber zu einem Ansteigen des Blutpegels führen, weshalb die Schutzvorrichtungen auch vorgesehen sind. Als Antikontaminations-Sperre werden üblicherweise hydrophobe Membranen verwendet. Diese sind gasdurchlässig, können aber von Blut nicht durchdrungen werden. Sie verhindern so bis zu einem gewissen Punkt, dass Blut in die Dialysemaschine gelangt. Steigt der Blutpegel nun bis zu der Membran und überschreitet der entstehende Druck einen Grenzwert, kann es zu einer Ruptur der Membran kommen. Kurze Zeit später kann dann Blut in die Dialysemaschine gelangen. Es ist deshalb wichtig, das Eindringen von Blut in die Wandler-Schutzvorrichtung und vor allem die Ruptur der Membran möglichst schnell zu erkennen.

Da die Fläche der Antikontaminationssperre bei handelsüblichen Wandler-Schutzvorrichtungen senkrecht zu den rohrförmigen Anschlüssen und damit nach Befestigung an der Behandlungsmaschine parallel zur Gehäuseoberfläche angeordnet ist, ist während der Behandlung für den Anwender nur noch eine Seite der Wandler-Schutzvorrichtung sichtbar.

Diese Anordnung (blutseitige Gehäuseoberfläche dem Nutzer zugewandt, blutabgewandte Gehäuseoberfläche dem Gerät zugewandt) verhindert eine schnelle Erkennung einer defekten Membran mit Blutdurchtritt (dies ist vor allem von der blutabgewandten Seite zu erkennen). Aufgabe der vorliegenden Erfindung ist es, eine Wandler-Schutzvorrichtung zur Verfügung zu stellen, die einfacher an der Dialysemaschine zu montieren ist.

Weiterhin soll die Erkennung einer drohenden oder erfolgten Membranruptur vereinfacht werden.

Nach der Lehre der vorliegenden Erfindung wird diese Aufgabe gelöst durch eine Wandler-Schutzvorrichtung nach Anspruch 1, ein Blutschlauchsystem nach Anspruch 12 und einer Anordnung nach Anspruch 13. Vorteilhafte Ausgestaltungen sind Gegenstand der Unteransprüche.

### Zusammenfassung der Erfindung

Die erfindungsgemäße Wandler-Schutzvorrichtung besteht aus einem flachen, transparenten Gehäuse mit einer Vorder- einer Rückwand und mindestens einer Seitenwand. Vorder- und Rückwand sind vorzugsweise gleich ausgebildet und austauschbar.

Vorder- und Rückwand bilden die Hauptfläche des Gehäuses. Die Fläche kann üblicherweise zwischen 1-5 cm² liegen. Die Seitenwand weist im Vergleich zu Vorder- und Rückwand eine kleinere Fläche auf. Der Abstand zwischen Vorder- und Rückwand kann zwischen 0,2 und 1 cm betragen, vorzugsweise 0,5 bis 0,8 cm.

Sind die Vorder- und Rückwand z.B. rund ausgebildet, werden sie durch eine umlaufende Seitenwand verbunden. Sind Vorder- und Rückwand z.B. rechteckig ausgebildet, so werden sie von vier Seitenwänden verbunden. Vorder- und Rückwand können beliebige Formen haben.

Das Gehäuse kann vorzugsweise aus zwei Teilen gebildet sein, die flüssigkeitsdicht zwischen den Hälften aufgrund der Trennung mit Hydrophobmembran, gasdicht zur Umgebung miteinander verbunden sind.

Weiterhin weist das Gehäuse eine gaspermeable Flüssigkeitssperre auf, die das Gehäuse in zwei Kammern aufgeteilt.

Die gaspermeable Flüssigkeitssperre kann dazu vorzugsweise zwischen den beiden flüssigkeitsdicht verbundenen Teilen des Gehäuses angeordnet sein.

Die gaspermeable Flüssigkeitssperre dient zum Schutz des Druckwandlers in der Dialysemaschine vor einem Eindringen von Flüssigkeit aus dem Schlauchsystem während des Betriebs. Bei der Flüssigkeit kann es sich um Blut, um Plasma oder Elektrolytlösungen, die im Schlauchsystem geführt werden, handeln.

Sie kann vorzugsweise auch die Sterilität des Schlauchsystems nach Entnahme aus der Verpackung gewährleisten, und damit eine Kontamination der Flüssigkeit, vorzugsweise des Blutes, verhindern.

Die gaspermeable Flüssigkeitssperre kann durch jedes Material gebildet werden, das durchlässig für Gas und undurchlässig für Flüssigkeiten ist. Vorzugsweise kann die gaspermeable Flüssigkeitssperre durch eine Hydrophobmembran gebildet werden. Weiterhin kann das Gehäuse Strukturen aufweisen, die zur Stützung der Hydrophobmembran dienen.

Die Wandler-Schutzvorrichtung umfasst weiterhin einen ersten rohrförmigen Verbinder zum Anschluss an eine Blutbehandlungsmaschine und einen zweiten rohrförmigen Verbinder zum Anschluss an ein Blutschlauchsystem. Jeweils ein rohrförmiger Verbinder ist mit jeweils einer Kammer des Gehäuses verbunden. Jeweils ein rohrförmiger Verbinder kann an jeweils einem Teil des Gehäuses angeordnet sein. Beide rohrförmigen Verbinder sind an der Seitenwand des flachen Gehäuses positioniert, so dass die rohrförmigen Verbinder und die Hauptfläche longitudinal ausgerichtet sind, wodurch Vorder- und Rückwand des Gehäuses als Grifffläche zur Montage an der Blutbehandlungsmaschine benutzbar sind und nach Montage Vorder- und Rückwand seitlich von vorne sichtbar sind.

Dabei kann es besonders vorteilhaft sein, wenn die beiden rohrförmigen Verbinder auf gegenüberliegenden Seiten des Gehäuses liegen.

Beide rohrförmigen Verbinder können nach dem Luer-Lock-System ausgebildet sein.

Vorzugsweise kann der erste rohrförmige Verbinder nach dem Luer Lock-System ausgebildet sein, wobei er ein Luer-Lock Verbinder des weiblichen Typs sein kann. In einer besonders bevorzugten Ausführungsform weist der konisch ausgebildete Verbinder ein Außengewinde auf.

Der zweite rohrförmige Verbinder kann in einer alternativen Ausführungsform auch als Schlauchsitz ausgebildet sein, in den das Blutschlauchsystem dann fest eingeklebt werden kann.

Die Erfindung betrifft weiterhin ein extrakorporales Blutschlauchsystem, das mindestens eine Wandler-Schutzvorrichtung nach Anspruch 1 bis 11 aufweist.

Die Erfindung betrifft weiterhin eine Anordnung aus einer Blutbehandlungsmaschine mit einem extrakorporalen Blutschlauchsystem nach Anspruch 12.

Eine beispielhafte Ausführungsform ist in den folgenden Figuren gezeigt.
Figur 1 zeigt schematisch einen extrakorporalen Kreislauf für eine therapeutische Behandlung nach dem Stand der Technik.
Figur 2 zeigt schematisch die Frontansicht einer an einer Behandlungsmaschine montierten Wandler-Schutzvorrichtung nach dem Stand der Technik.
Figur 3 zeigt schematisch die Seitenansicht einer an einer Behandlungsmaschine montierten Wandler-Schutzvorrichtung nach dem Stand der Technik.
Figur 4 zeigt schematisch die Seitenansicht einer an einer Behandlungsmaschine montierten, erfindungsgemäßen Wandler-Schutzvorrichtung.
Figur 5 zeigt schematisch den Längsschnitt einer erfindungsgemäßen Wandler-Schutzvorrichtung.

### Beschreibung

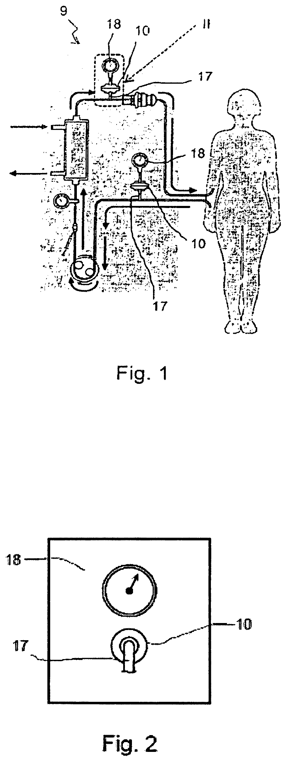

In Figur 1 ist schematisch ein extrakorporaler Blutkreislauf 9 gezeigt. Dabei ist sowohl im venösen als auch im arteriellen Zweig eine Druckwandler-Schutzvorrichtung 10 vorgesehen, die mit einem Druckwandler 18 einer Blutbehandlungsmaschine verbunden ist.

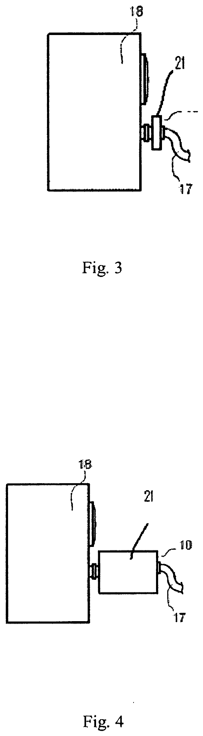

Figuren 2 und 3 zeigen eine Druckwandler-Schutzvorrichtung 10 nach dem Stand der Technik. Nach der Montage an die Behandlungsmaschine ist nur die Vorderseite der Druckwandler-Schutzvorrichtung von vorne zu erkennen, wobei ein Teil dieser Vorderseite durch die Blutschlauchleitung verdeckt ist. Da die Rückseite der Vorrichtung zur Maschine zeigt, ist eine Ruptur der Flüssigkeitssperre in der Schutzvorrichtung für den Benutzer schwer zu erkennen. In der Seitenansicht in Figur 3 ist die schmale Grifffläche 21 dieser Druckwandler-Schutzvorrichtung zu erkennen.

In Figur 4 ist eine erfindungsgemäße Druckwandler-Schutzvorrichtung 10 gezeigt. Hier sind beide Seiten seitlich von vorne zu erkennen. Sie bietet eine große Grifffläche 21, mittels derer man die Druckwandler-Schutzvorrichtung einfach an der Behandlungsmaschine montiert werden kann.

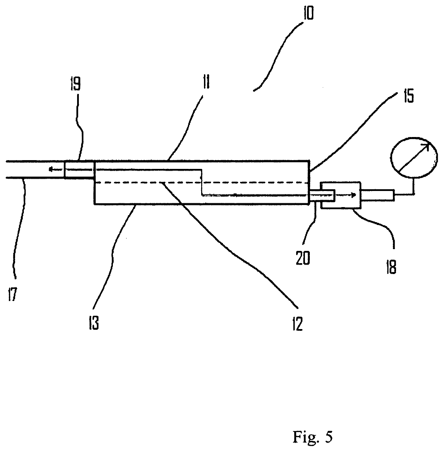

Figur 5 zeigt schematisch Längsschnitt einer erfindungsgemäßen Druckwandler-Schutzvorrichtung. Zwischen der Vorderwand 11 und der Rückwand 13 ist die gaspermeable Flüssigkeitssperre 12 angeordnet. Die gaspermeable Flüssigkeitssperre 12 kann zusätzlich durch Stützstrukturen, z. B. Stege (nicht gezeigt) stabilisiert sein. An der Seitenwand 15 sind die rohrförmigen Verbinder 19 und 20 angeordnet. Der rohrförmige Verbinder 19 ist mit dem Blutschlauchsystem 17 verbunden, der rohrförmige Verbinder 20 mit dem Druckwandler 18 in der Behandlungsmaschine.

## Patentansprüche

1. Wandler-Schutzvorrichtung (10) umfassend ein flaches, transparentes Gehäuse aus einer Vorder- und einer Rückwand (11, 13), die die Hauptfläche des Gehäuses bilden, mindestens einer schmalen Seitenwand (15) und einer gaspermeablen Flüssigkeitssperre (12), die das Gehäuse in zwei Kammern aufteilt und im Wesentlichen parallel zur Hauptfläche des Gehäuses angeordnet ist, weiterhin umfasst die Wandler-Schutzvorrichtung (10) einen ersten rohrförmigen Verbinder (19), zum Anschluss an eine Blutbehandlungsmaschine, und einen zweiten rohrförmigen Verbinder (20), zum Anschluss an ein Blutschlauchsystem (9), wobei jeweils ein rohrförmiger Verbinder (19, 20) mit jeweils einer Kammer verbunden ist, **dadurch gekennzeichnet, dass** die rohrförmigen Verbinder an der Seitenwand des flachen Gehäuses positioniert sind, wodurch die rohrförmigen Verbinder (19, 20) und die Hauptfläche des Gehäuses im Wesentlichen parallel entlang einer Achse ausgerichtet sind, sodass zur Montage an der Blutbehandlungsmaschine Vorder- und Rückwand (11, 13) des Gehäuses als Grifffläche benutzbar sind .

2. Vorrichtung (10) nach Anspruch 1 **dadurch gekennzeichnet, dass** das Gehäuse aus einem ersten und einem zweiten Teil besteht, beide Teile flüssigkeitsdicht miteinander verbunden sind und zwischen den Teilen die gaspermeable Flüssigkeitssperre (12) angeordnet ist.

3. Vorrichtung (10) nach Anspruch 1 oder 2, **dadurch gekennzeichnet, dass** es sich bei der gaspermeablen Flüssigkeitssperre (12) um eine Hydrophobmembran handelt.

4. Vorrichtung (10) nach Anspruch 1 bis 3, **dadurch gekennzeichnet, dass** das Gehäuse eine Struktur zur Stützung der Hydrophobmembran aufweist.

5. Vorrichtung (10) nach Anspruch 1 oder 2, **dadurch gekennzeichnet, dass** sich die beiden rohrförmigen Verbinder (19, 20) auf gegenüberliegenden Seiten des Gehäuses befinden.

6. Vorrichtung (10) nach Anspruch 1, 2 oder 5, **dadurch gekennzeichnet, dass** jeweils ein rohrförmiger Verbinder (19, 20) an jeweils einem Teil des Gehäuses angeordnet ist.

7. Vorrichtung (10) nach Anspruch 1, 2, 5 oder 6, **dadurch gekennzeichnet, dass** ein rohrförmiger Verbinder (19, 20) nach dem Luer-Lock-System ausgebildet ist.

8. Vorrichtung (10) nach Anspruch 1, 2, 5, 6 oder 7 **dadurch gekennzeichnet, dass** beide rohrförmige Verbinder (19, 20) nach dem Luer-Lock-System ausgebildet sind.

9. Vorrichtung (10) nach Anspruch 1, 2, 5, 6, oder 7, **dadurch gekennzeichnet, dass** ein rohrförmiger Verbinder (19, 20) einen Schlauchsitz aufweist.

10. Vorrichtung(10) nach einem der vorangehenden Ansprüche, **dadurch gekennzeichnet, dass** nach Montage der Wandler-Schutzvorrichtung an die Blutbehandlungsmaschine die Oberfläche derselben transversal zur Längsachse der Wandler-Schutzvorrichtung orientiert ist.

11. Vorrichtung (10) nach einem der vorangehenden Ansprüche, **dadurch gekennzeichnet, dass** das Gehäuse aus Kunststoff, z.B. Polypropylen, Polycarbonat, PVC oder Polysulfon, besteht.

12. Extrakorporales Blutschlauchsystem (9) mit mindestens einer Wandler-Schutzvorrichtung (10) nach einem der Ansprüche 1 bis 11.

13. Anordnung aus Blutbehandlungsmaschine mit einem extrakorporalen Blutschlauchsystem (9) nach Anspruch 12.

## Claims

1. A transducer protective device (10) comprising a flat transparent housing, consisting of a front wall and a back wall (11, 13), which form the main surface of the housing, at least one narrow side wall (15) and one gas-permeable liquid barrier (12), dividing the housing into two chambers and arranged essentially parallel to the main surface of the housing; in addition, the transducer protective device (10) comprises a first tubular connector (19) for connection to a blood treatment machine, and a second tubular connector (20) for connection to a blood tubing system (9), wherein a tubular connector (19, 20) is connected to each chamber,
**characterized in that**
the tubular connectors are positioned on the side wall of the flat housing, so that the tubular connectors (19, 20) and the main surface of the housing are aligned essentially in parallel along an axis, so that the front wall and the back wall (11, 13) of the housing can be used as handle surfaces for mounting the device on the blood treatment machine.

2. The device (10) according to claim 1,
**characterized in that**
the housing consists of a first part and a second part, the two parts being connected to one another in a fluid-tight connection, and the gas-permeable liquid barrier (12) being arranged between the parts.

3. The device (10) according to claim 1 or 2,
**characterized in that**
the gas-permeable liquid barrier (12) is a hydrophobic membrane.

4. The device (10) according to claims 1 through 3,
**characterized in that**
the housing has a structure for supporting the hydrophobic membrane.

5. The device (10) according to claim 1 or 2,
**characterized in that**
the two tubular connectors (19, 20) are situated on opposite sides of the housing.

6. The device (10) according to claims 1, 2 or 5,
**characterized in that**
one tubular connector (19, 20) is arranged on each part of the housing.

7. The device (10) according to claims 1, 2, 5 or 6,
**characterized in that**
one tubular connector (19, 20) is designed according to the Luer-lock system.

8. The device (10) according to claims 1, 2, 5, 6 or 7,
**characterized in that**
the two tubular connectors (19, 20) are designed according to the Luer-lock system.

9. The device (10) according to claims 1, 2, 5, 6 or 7,
**characterized in that**
a tubular connector (19, 20) has a tubular seating.

10. The device (10) according to any one of the preceding claims,
**characterized in that**
after mounting the transducer protective device on the blood treatment machine, the surface of same is oriented transversely to the longitudinal axis of the transducer protective device.

11. The device (10) according to any one of the preceding claims,
**characterized in that**
the housing is made of plastic, e.g., polypropylene, polycarbonate, PVC or polysulfone.

12. An extracorporeal blood tubing system (9) having at least one transducer protective device (10) according to any one of claims 1 to 11.

13. An arrangement of a blood treatment machine having an extracorporeal blood tubing system (9) according to claim 12.

## Revendications

1. Dispositif de protection de convertisseur (10) comprenant un boîtier transparent plat composé d'une paroi avant et d'une paroi arrière (11,13) qui constituent la surface principale du boîtier, d'au moins une paroi latérale étroite (15) et d'une barrière anti-liquide perméable au gaz (12) qui divise le boîtier en deux chambres et est disposée sensiblement parallèlement à la surface principale du boîtier, le dispositif de protection de convertisseur (10) comprenant en outre un premier connecteur de forme tubulaire (19) destiné à être raccordé à une machine de traitement sanguin, et un second connecteur de forme tubulaire (20) destiné à être raccordé à un système de tuyau sanguin (9), respectivement un connecteur de forme tubulaire (19,20) étant raccordé respectivement à une chambre, **caractérisé en ce que** les connecteurs de forme tubulaires sont positionnés au niveau de la paroi latérale du boîtier plat, ce qui fait que les connecteurs tubulaires (19,20) et la surface principale du boîtier sont orientés sensiblement parallèlement le long d'un axe, de sorte que, pour le montage à la machine de traitement sanguin, les parois avant et arrière (11,13) du boîtier sont utilisables comme surface de préhension.

2. Dispositif (10) selon la revendication 1, **caractérisé en ce que** le boîtier est composé d'une première et d'une seconde pièce, les deux pièces étant connectées entre elles de manière étanche au liquide et la barrière anti-liquide perméable aux gaz (12) étant disposé entre les pièces.

3. Dispositif (10) selon la revendication 1 ou 2, **caractérisé en ce que** la barrière anti-liquide perméable aux gaz (12) est une membrane hydrophobe.

4. Dispositif (10) selon les revendications 1 à 3, **caractérisé en ce que** le boîtier présente une structure de soutien de la membrane hydrophobe.

5. Dispositif (10) selon la revendication 1 ou 2, **caractérisé en ce que** les deux connecteurs de forme tubulaire (19,20) se trouvent sur des faces opposées du boîtier.

6. Dispositif (10) selon la revendication 1, 2 ou 5, **caractérisé en ce qu'**un connecteur de forme tubulaire (19,20) est respectivement disposé respectivement au niveau d'une pièce du boîtier.

7. Dispositif (10) selon la revendication 1, 2, 5 ou 6, **caractérisé en ce qu'**un connecteur de forme tubulaire (19,20) est respectivement réalisé suivant le système luer lock.

8. Dispositif (10) selon la revendication 1, 2, 5, 6 ou 7, **caractérisé en ce que** les deux connecteurs de forme tubulaire (19,20) sont réalisés suivant le système luer lock.

9. Dispositif (10) selon la revendication 1, 2, 5, 6 ou 7, **caractérisé en ce qu'**un connecteur de forme tubulaire (19,20) présente un siège tubulaire.

10. Dispositif (10) selon une des revendications précédentes, **caractérisé en ce que**, après le montage du dispositif de protection de convertisseur à la machine de traitement sanguin, sa surface est orientée transversalement à l'axe longitudinal du dispositif de protection de convertisseur.

11. Dispositif (10) selon une des revendications précédentes, **caractérisé en ce que** le boîtier est composé de plastique, par exemple de polypropylène, de polycarbonate, de PVC ou de polysulfone.

12. Système de tuyau sanguin extracorporel (9) comportant au moins un dispositif de protection de convertisseur (10) selon une des revendications 1 à 11.

13. Agencement composé d'une machine de traitement sanguin doté d'un système de tuyau sanguin extracorporel (9) selon la revendication 12.
